# EUROPEAN PATENT APPLICATION

(11) **EP 1 360 970 A1**
(43) Date of publication of application: **12.11.2003**
(21) Application number: 03014681.5
(22) Date of filing: 26.08.1999
(51) Int. Cl.: A61M 5/32, A61M 5/50

(54) **Method for using a safety shield assembly and related combinations thereof**

(30) Priority: 28.08.1998 US 98287 P
(62) Divisional of application: 99116669.5
(71) Applicant: Becton, Dickinson and Company, Franklin Lakes, New Jersey 07417-1880 (US)
(72) Inventor: Newby, Mark C., Tuxedo, New York 10987 (US); Bennett, Michael C., Summit, New Jersey 07901 (US); Crawford, Jamie, New York, New York 10025 (US)
(74) Representative: Selting, Günther, Dipl.-Ing.

(57) **Abstract**

The present invention is a method of using a safety shield assembly having a shield and a collar and more particularly a method of using a safety shield assembly with a fluid handling device whereby the shield may be pivoted with respect to the collar. Preferably, a method of using a safety shield assembly with a needle assembly, an intravenous infusion set a syringe, a catheter or other fluid handling devices or assemblies that contain piercing elements.

## Description

The present invention relates to a shield for a needle and more particularly to a safety shield assembly that may be used in conjunction with a syringe assembly, a hypodermic needle, a needle assembly, a needle assembly with a needle holder, a blood collection needle, a blood collection set, an intravenous infusion set or other fluid handing devices or assemblies that contain piercing elements.

### Description of Related Art

Disposable medical devices having piercing elements for administering a medication or withdrawing a fluid, such as hypodermic needles, blood collecting needles, fluid handling needles and assemblies thereof, require safe and convenient handling. The piercing elements include, for example, pointed needle cannula or blunt ended cannula.

Safe and convenient handling of disposable medical devices is recognized by those in the medical arts so as to minimize exposure to blood borne pathogens. Safe and convenient handling of disposable medical devices results in the disposal of the medical devices intact.

As a result of this recognition, numerous devices have been developed for shielding needles after use. Many of these devices are somewhat complex and costly. In addition, many of these devices are cumbersome to use in performing procedures. Furthermore, some of the devices are so specific that they preclude use of the device in certain procedures or with certain devices and/or assemblies:

Therefore, there exists a need for a safety shield assembly: (i) that is easily manufactured; (ii) that is applicable to many devices; (iii) that is simple to use with one hand; (iv) that can be safely disposed of; (v) that does not interfere with normal practices of needle use; (vi) that has tactile features whereby the user may be deterred from contacting the needle, the user may easily orient the needle with the patient and easily actuate and engage the shield assembly; (vii) that has visual features whereby the user may be deterred from contacting the needle, the user may easily orient the needle with the patient and easily actuate and engage the shield assembly; (viii) that is not bulky; (ix) that includes means for minimizing exposure to the user of residual fluid leaking from the needle; and (x) provides minimal exposure to the user because the needle shield is immediately initiated by the user after the needle is withdrawn from the patient's vein.

US-A-5,445,619 and US-A-5,662,617 disclose needle assemblies as defined in the introductory part of claim 1. The needle assembly has a needle and a safety shield assembly comprising a slot and being adapted to be moved from a position in which the intravenous end of the needle is exposed to a position at which the intravenous end is shielded within the slot of the shield. The orientation of the needle is such that the bevel down orientation faces the slot of the shield.

### SUMMARY OF THE INVENTION

It is an object of the invention to provide a needle assembly enabling the user to easily orient the needle with the patient and easily actuate and engage the shield assembly.

The assembly of the present invention is defined by claim 1.

The assembly of the present invention preferably comprises, a shield, means for connecting the shield to a fluid handling device that contains a piercing element such as needle, means for pivoting the shield away from the needle, means for securely covering and/or containing the needle within the shield and means for securely locking the shield in a final non-retractable closed position over the needle.

Preferably, the shield comprises a rearward end, a forward end, a slot or longitudinal opening for housing the used needle in the forward end, means for securing the needle in the slot, means for guiding the needle into the slot, means for connecting the shield and the fluid handling device, means for guiding the user's fingers to move the shield into various positions; and means for retaining the shield securely over the used needle.

Desirably, the means for connecting the shield to the fluid handling device is with a collar. Preferably, the shield is movably connected to a collar which is connected to a fluid handling device.

Preferably, the shield is connected to the collar by a hanger bar that engages with a hook arm that is located on the collar so that the shield may be pivoted with respect to the collar and the shield is able to easily move into several positions. It is within the purview of the present invention to include any structure for connecting the shield to the collar so that the shield may be pivoted with respect to the collar. These structures include-known mechanical hinges and various linkages, living hinges, or combinations of hinges and linkages.

Most preferably, the shield is connected to the collar by an interference fit between the hanger bar and the hook bar. Therefore, the shield is always oriented in a stable position and will not move forward or backwards unless movement of the shield is relative to the hanger bar and the hook bar is positively initiated by the user.

Alternatively, it is within the purview of the present invention that the shield and collar is unitary one-piece structure may be accomplished by many methods including molding the shield and the collar as a one-piece unit thereby eliminating the need to separately assemble the shield and the collar during the manufacturing process.

The assembly of the present invention may further comprise tactile and visual means for deterring the user from contacting the needle, providing easy orientation of the needle with the patient and provide the user with a guide for actuation and engagement with the shield.

The assembly of the present invention may further comprise means for minimizing exposure by the user to residual fluid leaking from a used needle, whereby a polymer material is located in the shield. Desirably, the material may be a gel like material.

Most desirably, the assembly of the present invention is such that the cooperating parts of the assembly provide the means for the shield to move into a forward use position over the needle. Thus, by simple movement of the shield into a positive position over the used needle, the assembly is ready for subsequent disposal. Therefore, the safety shield assembly of the present invention provides minimal exposure of the user to a needle because the shield is immediately initiated by the user after the needle is withdrawn from the patient's vein.

Desirably, the assembly of the present invention may be used with a syringe assembly, a hypodermic needle, a needle assembly, a needle assembly with a needle holder, a blood collection set, an intravenous infusion set or other fluid handling devices. Preferably, the assembly of the present invention is used with a needle assembly comprising a needle and a hub. Preferably the needle is a conventional double ended needle.

Most preferably, the present invention is used with a needle assembly comprising a hub and a needle connected to the hub whereby the needle comprises a non-patient end and an intravenous end. The collar of the present invention is connected to the hub, which comprises a hook arm and the shield is movably connected to the hook arm whereby the shield may be pivoted with respect to the collar and easily moved into several positions.

Preferably, the collar is fitted with the hub of the needle assembly whereby the collar cannot rotate around the hub. Additionally, the collar includes cooperating means that mate with reciprocal means on the shield to lock the shield in a final closed position .

Alternatively, it is within the purview of the present invention that the collar and hub is a unitary one-piece structure. The one piece structure may be accomplished by many methods including molding the collar and the hub as a one-piece unit thereby eliminating the need to separately assemble the collar to the hub during the manufacturing process.

Most preferably, the collar is fitted with the hub of the needle assembly whereby the bevel surface or bevel up surface of the intravenous end of the needle faces the same side of the collar when the shield is in the open position. Alignment of the collar, hub, shield and needle with the bevel surface up makes it easier to insert the needle into the patient without manipulating the assembly. The orientation of the intravenous end of the needle as bevel up assures the user that the needle is properly oriented for use and does not require any manipulation before use. Most notably, the orientation of the shield provides a visual indication to the user of the orientation of the bevel surface of the needle.

Preferably, the shield is capable of pivoting from an open position where the intravenous end of the needle is exposed and bevel up, to an intermediate-position where the needle is partially covered, to a final closed nonretractable position where the needle is completely covered and the shield is locked and no longer able to be move out of the closed position.

Alternatively, it is within the purview of the present invention that the shield, collar and hub is a unitary one-piece structure. The one-piece structure may be accomplished by many methods including molding the shield, collar and hub as a one-piece unit thereby eliminating the need to separately assemble the shield, collar and hub during the manufacturing process.

It is an advantage of the present invention that the shield covering the used intravenous end of the needle provides easy containment of the used needle. A further advantage of the shield is that it will not only move upon initiation by the user.

The assembly of the present invention when used with a fluid handling device is also easily disposable when removed from a conventional needle holder, or other such device.

Another important feature of the present invention includes means for locking the shield in a closed permanent position covering the needle. The closed permanent position will generally withstand the normal forces encountered during proper disposal of the safety shield assembly when it is removed from a conventional needle holder.

A notable attribute of the present invention is that it is easily adaptable with many devices. For example, with syringe assemblies, hypodermic needles, needle holders, blood collection needles, blood collection sets, intravenous infusion sets such as catheters or other fluid handling devices or assemblies that contain piercing elements.

Another notable attribute of the present invention is that the tactile and visual features deter the user from touching the needle, allow the user to easily orient the needle with the patient and guide the user to actuate and engage the shield of the assembly.

### DESCRIPTION OF THE DRAWINGS

FIG. 1 is a perspective view of the safety shield assembly of the present invention as connected to a needle assembly and related packaging features.
FIG. 2 is a perspective view of the unassembled pieces of FIG. 1.
FIG. 3 is a bottom view of the shield as shown in FIG. 2.
FIG. 4 is a cross sectional view of the collar as shown in of FIG. 2 taken along lines 4-4 thereof.
FIG. 5 is a cross sectional view of the needle hub as shown in FIG. 2 taken along lines 5-5 thereof.
FIG. 6 is a cross sectional view of the shield of FIG. 2 taken along lines 6-6 thereof.
FIGS. 7-11 illustrate the use of the safety shield assembly with the needle assembly of FIG. 1 with a conventional needle holder.
FIG. 12 is a cross sectional view of the assemblies in use with a conventional needle holder as shown in FIG. 9 taken along lines 12-12 thereof.
FIG. 13 is a cross sectional view of the assemblies in use with a conventional needle holder as shown in FIG. 11 taken along lines 13-13 thereof.
FIG. 14 is a cross-sectional view of the assemblies of FIG. 11 taken along lines 14-14 thereof.
FIG. 15 is a bottom view of the assemblies as shown in FIG. 11.
FIG. 16 illustrates an additional embodiment of the present invention, whereby a gel material is located in the shield as shown in a bottom view of the assemblies of FIG. 11.
FIG. 17 is a perspective view of an additional embodiment of the present invention in use with a blood collection set.
FIG. 18 is a perspective view of an additional embodiment of the present invention in use with a syringe.
FIG. 19 is a perspective view of an additional embodiment of the present invention in use with a catheter.

### DETAILED DESCRIPTION

While this invention is satisfied by embodiments in many different forms, there is shown in the drawings and will herein be described in detail, the preferred embodiments of the invention, with the understanding that the present disclosure is to be considered as exemplary of the principles of the invention and is not intended to limit the invention to the embodiments illustrated. Various other modifications will be apparent to and readily made by those skilled in the art without departing from the scope and spirit of the invention. The scope of the invention will be measured by the appended claims and their equivalents.

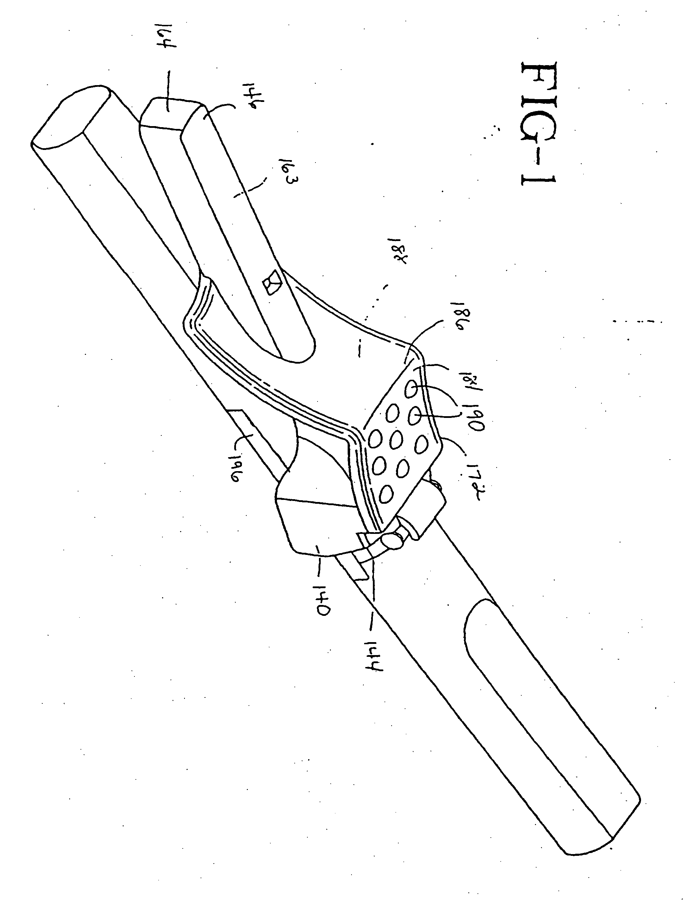

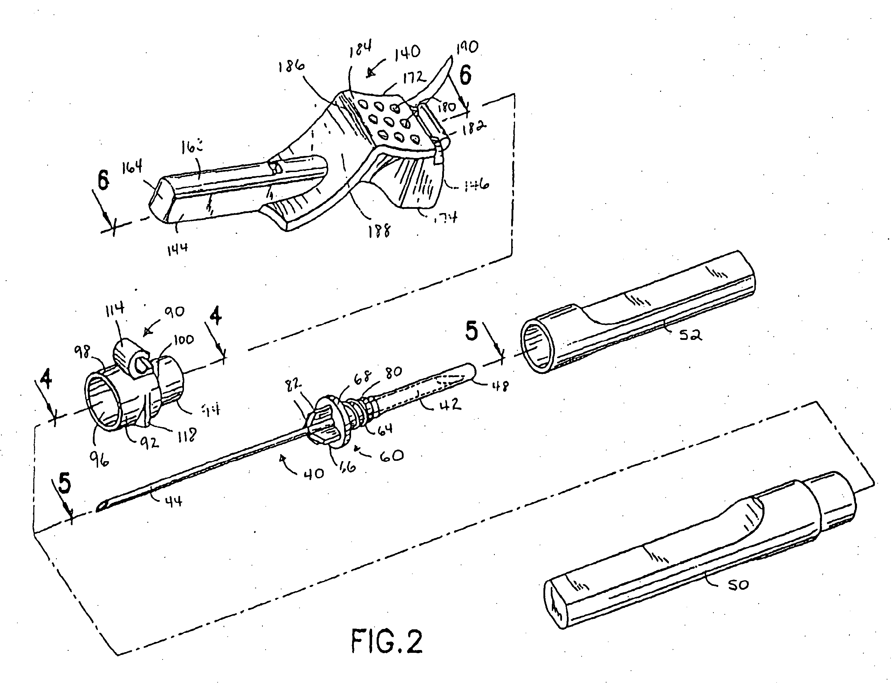

Referring to the drawings in which like reference characters refer to like parts throughout the several views thereof, FIGS. 1 and 2 illustrate a needle assembly with the safety shield assembly of the present invention and the related packaging features. The needle assembly includes a needle **40** and a hub **60** and packaging features to cover the needle and a label and the safety shield assembly includes a collar **90** and a shield **140.**

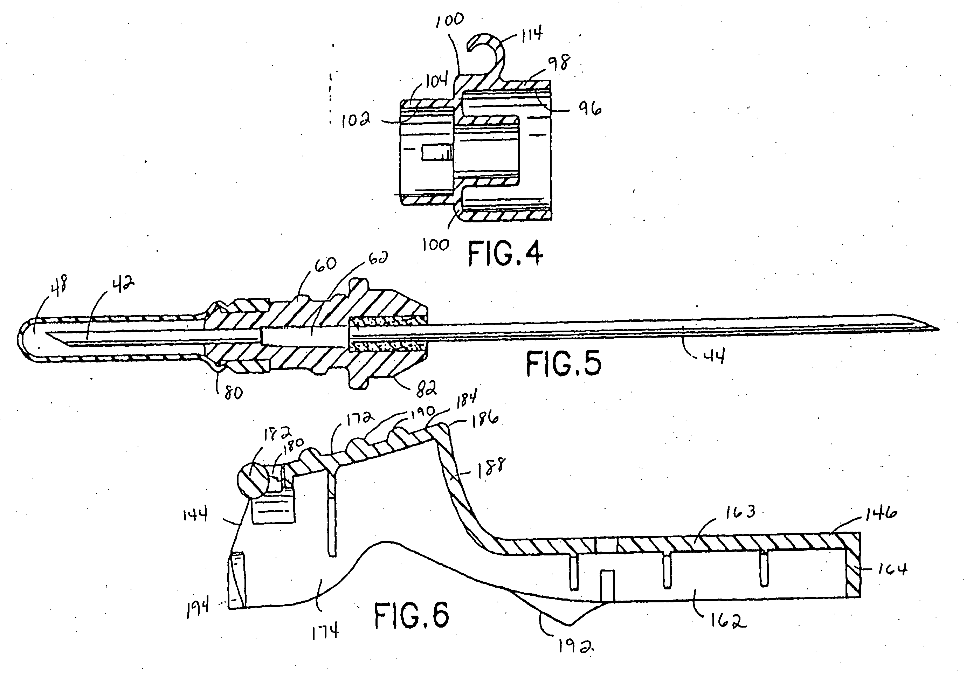

As shown in FIG. 2 and 5, needle **40** includes a non-patient end **42,** an intravenous end **44** and a passageway **46** extending between the non-patient end and the intravenous end. An elastomeric sleeve **48** covers the non-patient end, a rigid sleeve **50** covers the intravenous end and a second rigid sleeve **52** covers the non-patient end and the elastomeric sleeve. As shown in FIG. 1, a label **196** may also be applied to the finally assembled parts.

As shown in FIGS. 2 and 5, hub **60** includes a threaded end **64,** a ribbed end **66** and passageway **62** extending between the threaded end and the ribbed end. Threaded end **64** and ribbed end **66** are separated by flange **68**. Non-patient end **42** of needle **40** extends from threaded end **64** and intravenous end **44** of needle **40** extends from ribbed end **66**. Preferably, threaded end **64** comprises male threads **80** for mounting the hub on a conventional needle holder and ribbed end **66** comprises male ribs **82** for connecting the hub and collar **90.**

As shown in FIGS. 2 and 4, collar **90** includes two sections, a forward annular skirt **92** and a rearward annular skirt **94.** The forward annular skirt is cylindrical comprising an inner sidewall **96** and an outer sidewall **98** and mates with the rearward annular skirt at a shoulder **100.** Rearward annular skirt **94** is cylindrical comprising an inner sidewall **102** and an outer sidewall **104** and extends from shoulder **100** opposite of forward annular skirt **92.** The inner diameter of forward annular skirt **92** is larger than the inner diameter of rearward annular skirt **94.** Alternatively, the inner diameters for collar **90** can be formed as a constant inner diameter of the same.

Extending on outer sidewall **98** of forward skirt section **92** is a hook member **114** and located opposite or downwardly of hook member **114** on outer sidewall **98** are locking dents or protrusions **118.**

As shown in FIGS. 2 and 6, shield **140** comprises a rearward end **144** and a forward end **146.**

Forward end **146** of shield **140** includes a slot or longitudinal opening **160** formed by sidewalls **162** that extend downwardly from top section **163** and run substantially opposite of one another in parallel along the length of slot **160** towards forward end sidewall **164.** Means for trapping a needle in slot **160** includes an arm **167** that is located at one of sidewalls **162** to secure the used needle.

Arm **167** is deflectable by the needle when the needle enters slot **160.** Once the needle passes the end of the arm, the arm moves back to its original position, whereby the needle is permanently trapped in slot **160** by arm **167**.

At rearward end **144** of the shield is a collar engaging area **166** that is a continuation of slot **160.** Collar engaging area **166** includes a rearward end **168,** a forward end **170,** a top finger guide area **172,** parallel sidewalls **174** that extend downwardly and inwardly from top finger guide area **172** and into sidewalls **162,** an underside area **176** for surrounding collar **90,** and extending arms **180** to hold hanger bar **182**. Parallel sidewalls **174** include an inner surface **175** where barb dents **194** are located.

Top finger guide area **172** comprises a first ramp **184** that extends slightly on an upwardly slope from the rearward end of the collar engaging area to a shoulder **186.** From shoulder **186** extends a second ramp **188** which slopes downwardly towards top section **163.** Most preferably, first ramp **184** comprises touch bumps **190.** The touch bumps provide a tactile and visual guide to alert the user that the user's finger has contacted the shield and that the shield is in a defined or controlled position. The touch bumps may be any configuration so long as they extend and are distinct from the top finger guide area. The touch bumps may also be of a distinguishing color as compared to the top finger guide area or the shield.

Second ramp **188** has interior surface **192** for urging the needle toward the center of slot **160** as the shield is being rotated into the closed position. The exterior surfaces are slightly inclined and extending radially from the second ramp. The interior surfaces are especially helpful if the longitudinal axis of the needle is misaligned with respect to the longitudinal axis of the hub.

Extending arms **180** are located at rearward end **168** and at the beginning of top finger area **172** and hold hanger bar **182.**

Located downwardly from extending arm **180** and hanger bar **182** and on inner surface **175** of parallel sidewalls **174** are barb dents **194.** The barb dents cooperate with locking dents **118** on collar **90** to secure the shield in its final locked position.

The safety shield assembly and the needle assembly are assembled together whereby needle **40** is connected to hub **60** and sealed with adhesive at the ends of the hub. Hub **60** is then joined with collar **90** by ultra-sonic welding techniques or any other bonding techniques, or mechanical fit, whereby rearward annular skirt **94** of collar **90** mates with ribbed end **66** of the hub. Male ribs **82** of the hub are contained or forced fitted within inner sidewall **102** of rearward annular skirt **94** of collar **90.** The collar is aligned with the intravenous end of the needle whereby the hook arm is aligned with the bevel up of the needle. Then rigid sleeve **50** is force fitted into inner side wall **96** of forward annular skirt **92** of collar **90** to cover the needle. Thereafter, shield **140** is connected to collar **90** whereby hanger bar **182** is force fitted into hook member **114** whereby slot **160** faces rigid sleeve **50.** Most preferably, the shield is connected to the collar by a force fit or interface fit between the hanger bar and the hook bar. Therefore, the shield is always oriented in a stable position and will not move unless movement of the shield is positively initiated by the user. To assemble the last piece, shield **140** is moved towards rigid sleeve **50** and second rigid sleeve **52** is force fitted onto outer sidewall **104** of rearward annular skirt **94** of collar **90**.

In addition, a label **196** may be applied to the finally assembled parts. The label may be used to prevent tamper resistance of the parts, so that they are not reused.

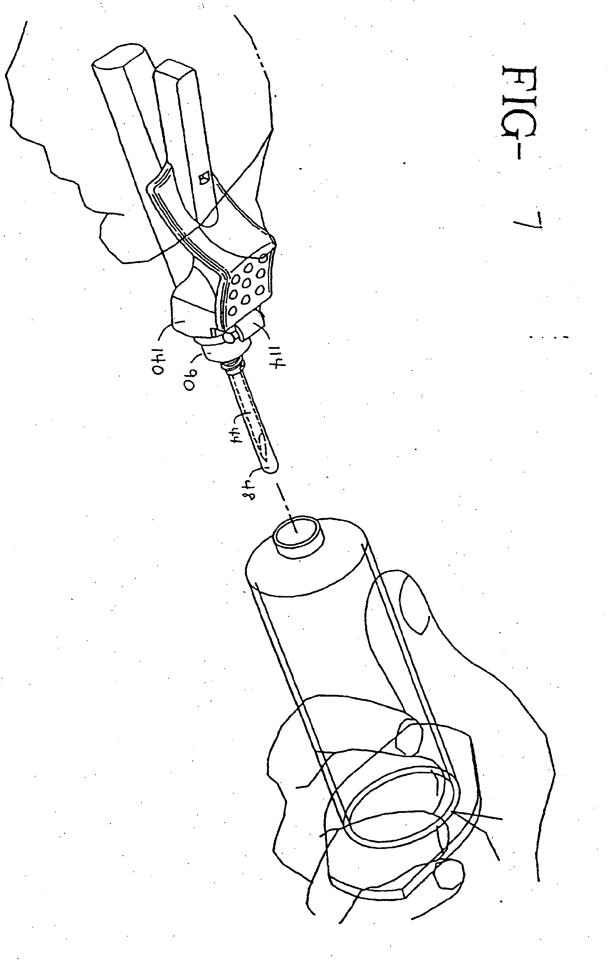

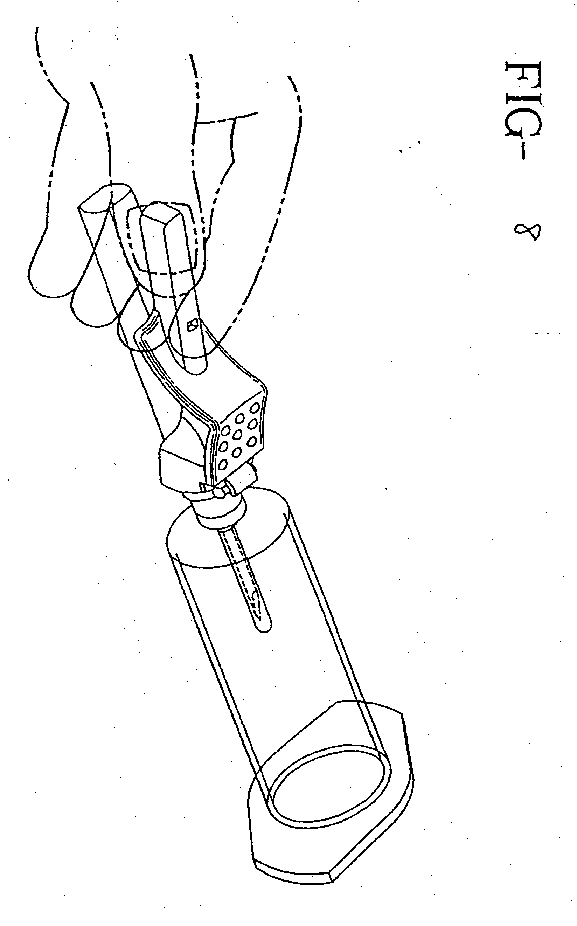

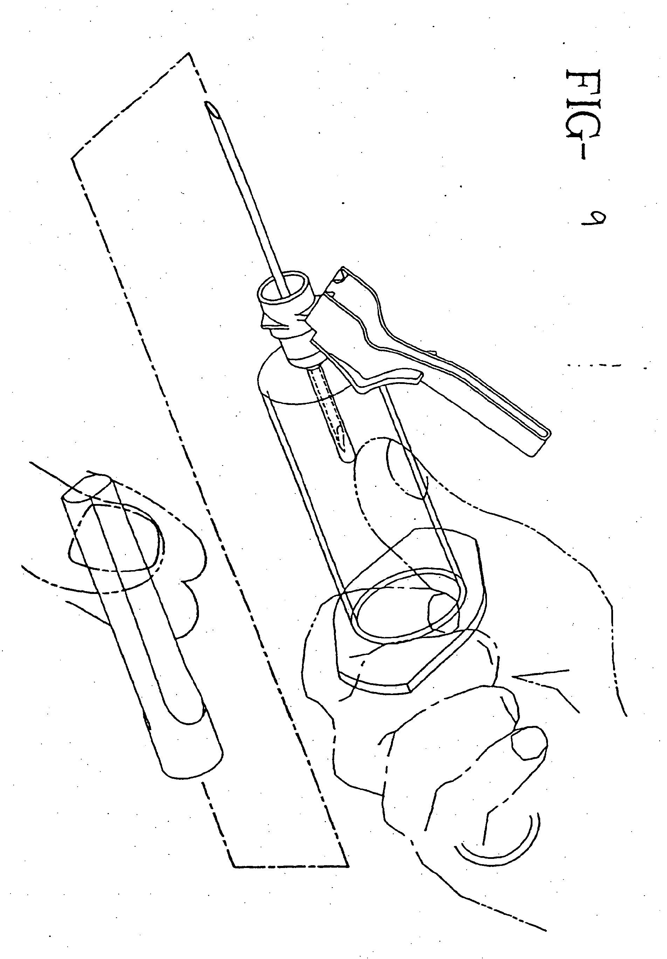

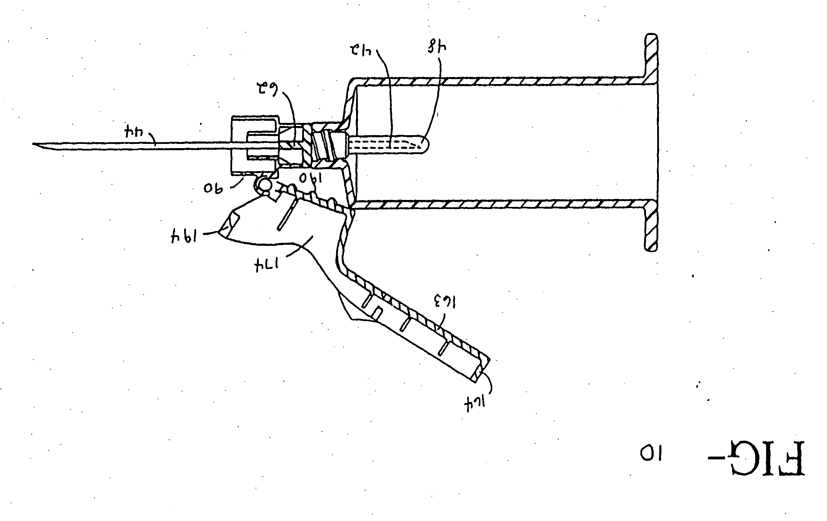

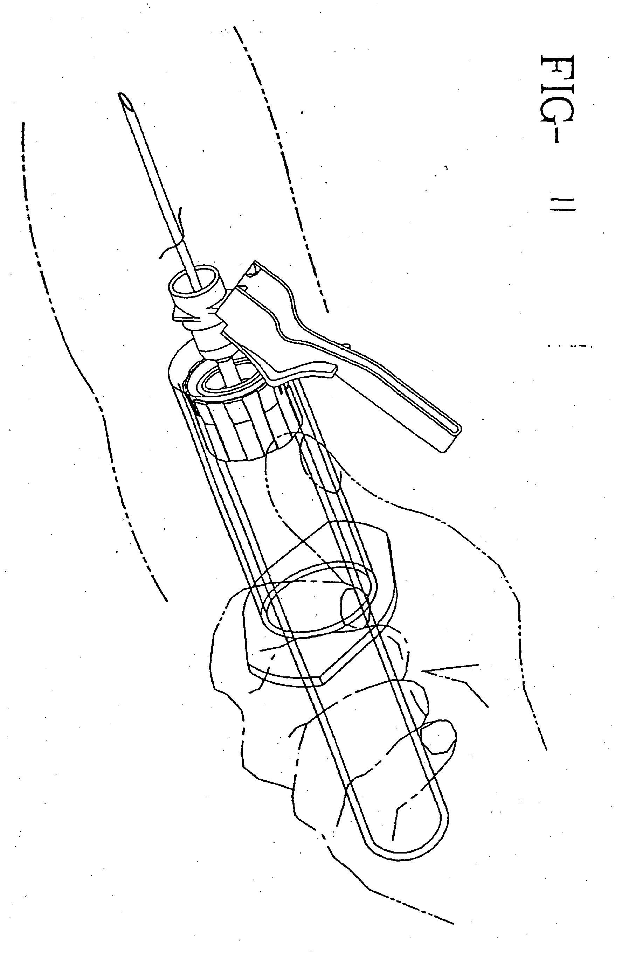

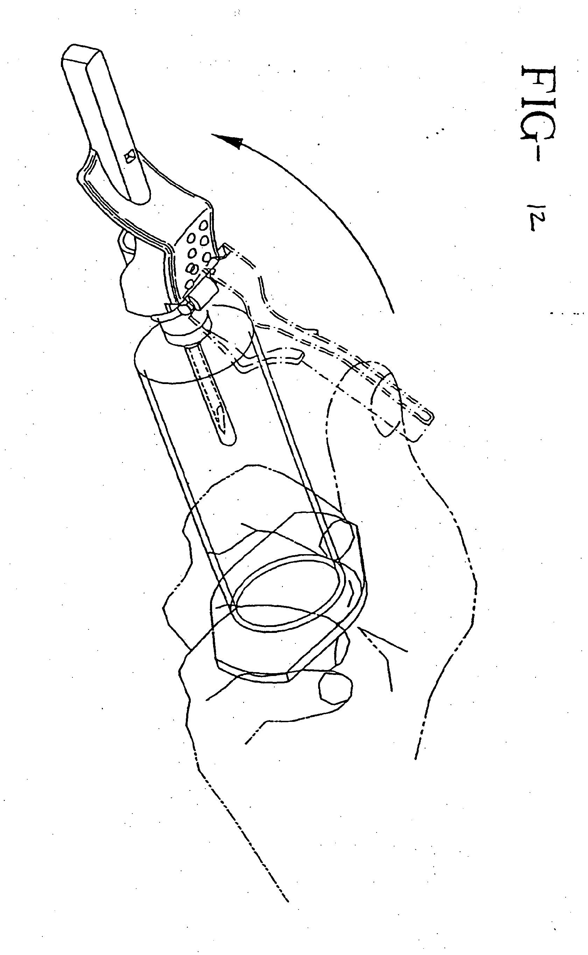

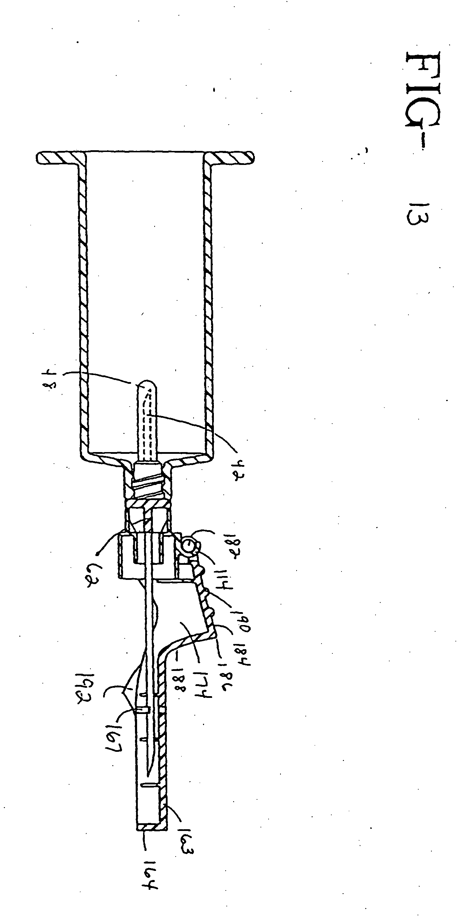

In use, as shown in FIGS. 7-15, the non-patient needle shield is removed and then a needle holder is screwed onto the hub of the needle. As specifically shown in FIGS. 8 and 12 the shield is then rotated back by the user towards the needle holder. Then as shown in FIG. 9, the intravenous needle shield is removed from covering the intravenous needle. Then as shown in FIG. 10, a venipuncture is conducted whereby the intravenous end of the needle is inserted into a vein of a patient and an evacuated tube having a closure is inserted into the needle holder. Then as shown in FIGS. 11 and 13, when the venipuncture is complete the user easily rotates the shield from the open position towards the intravenous needle to an intermediate position and then the user pushes on the shield at the top finger guide area to move the shield into a final, non-retractable locked position whereby the needle is trapped in the longtiudinal opening, within the arm of the needle shield and barb dents **184** of the shield are held by locking dents **118** of collar **90.** As the shield is pivoted the barb dents deflect over and are held by the locking dents.

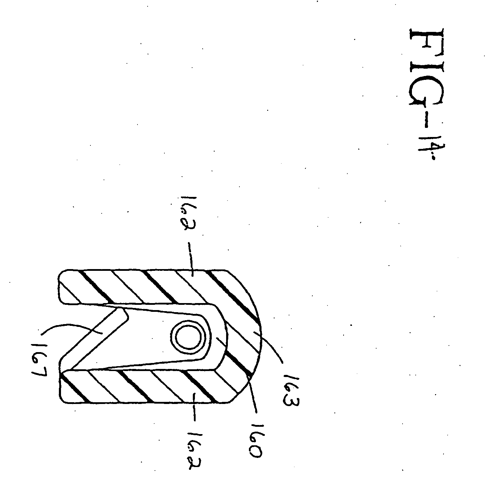

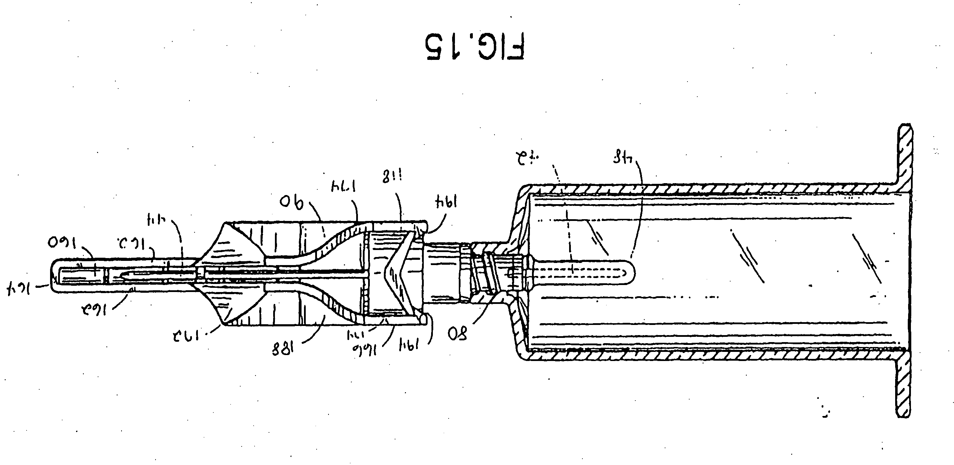

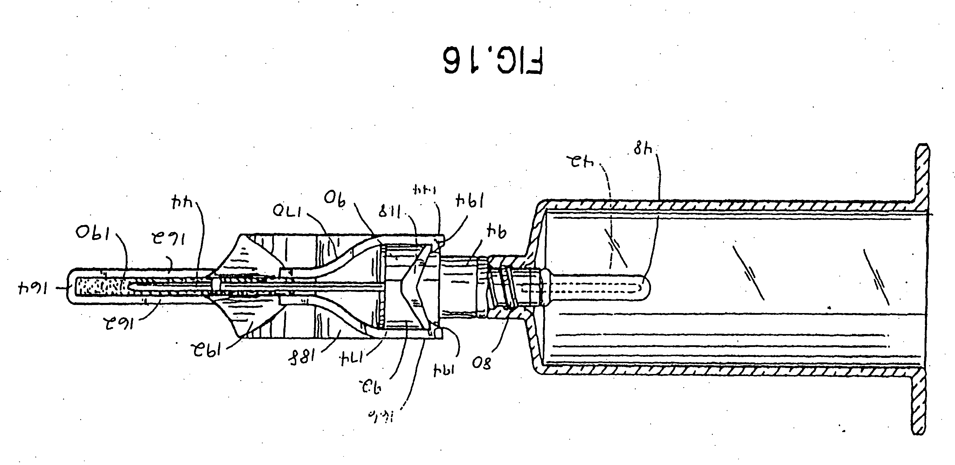

The needle is contained within the shield as the shield is pivoted into the closed position, whereby the needle snaps past arm **167** and is trapped as shown in FIGS. 14 and 15. Alternatively as shown in FIG. 16, a gel material **190** is located in the shield near arm **167** so that when the needle snaps past arm **167** it will come to rest in gel material **190**. The gel material will contain any residual fluid that may be on the needle.

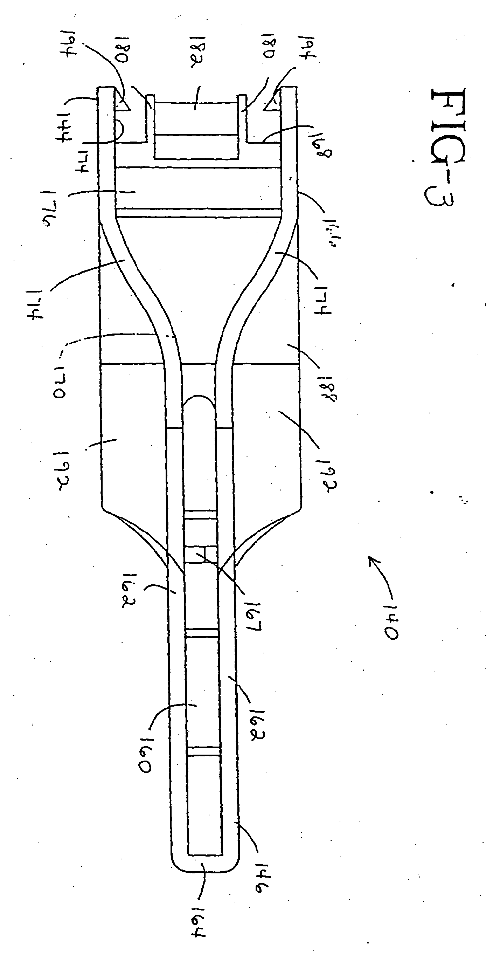

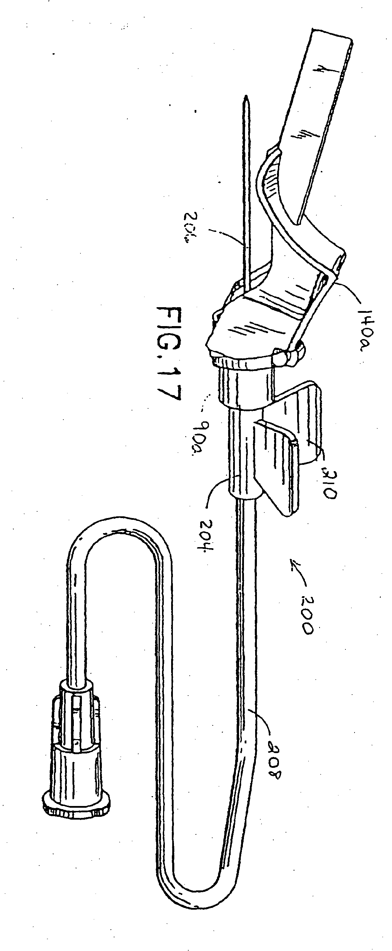

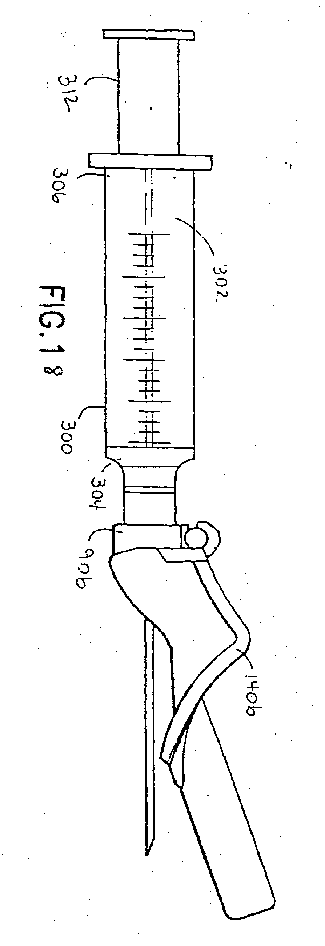

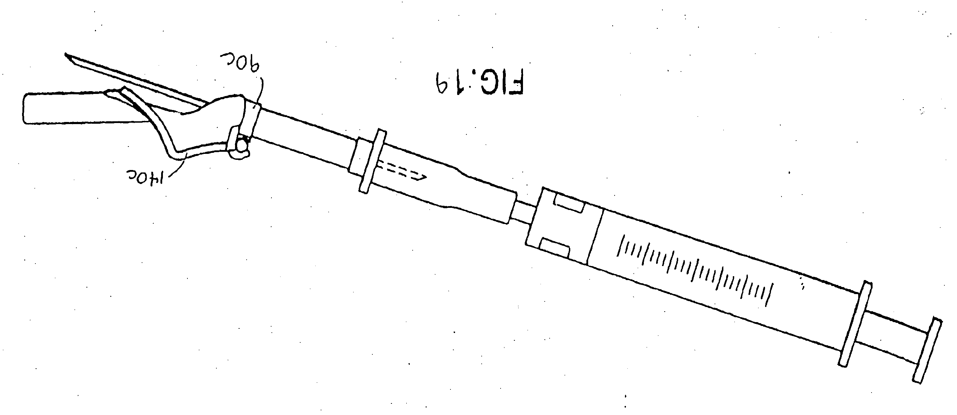

FIGS. 17, 18, and 19 are further embodiments of the invention that include may components which are substantially identical to the components of FIGS. 1-3. Accordingly, similar components performing similar functions will be numbered identically to those components of FIGS. 1-3, except that a suffix "a" will be used to identify those similar components in FIG. 17, a suffix "b" will be used to identify those similar components in FIG. 18 and a suffix "c" will be used to identify those similar components in FIG. 19.

Alternatively, the safety shield assembly of the present invention may be used in conjunction with a conventional intravenous (IV) fusion set, as illustrated in FIG. 17.

For purposes of illustration, shield **140a** and collar **90a** are connected to a conventional IV infusion set, **200,** or butterfly structure comprising a needle body-with. a needle hub **204** extending from the forward end of the needle body and a needle **206** embedded in hub **204.** Extending from the rearward end of the needle body is flexible tubing **208** which is conventional and utilized to allow the user to manipulate the structure and to connect it subsequently to supplies of infusion liquids or for the return of collected blood if the arrangement is being used to collect blood.

Infusion set **200** further comprises flexible wings **210** attached to and projecting outwardly from needle hub **204**.

Alternatively, the safety shield assembly of the present invention may be used inconjunction with a syringe, as illustrated in FIG. 18.

For purposes of illustration, shield **140b** and collar **90b** are connected to a conventional hypodermic syringe **300** comprising a syringe barrel **302** having a distal end **304** a proximal end **306** and a plunger **312.**

Alternatively, the present invention may be used in conjunction with a catheter as illustrated in FIG. 19.

The shield and collar of the safety shield assembly of the present invention are comprised of moldable parts which can be mass produced from a variety of materials including, for example, polyethylene, polyvinyl chloride, polystryene or polyethylene and the like. Materials will be selected which will provide the proper covering and support for the structure of the invention in its use, but which will provide also a degree of resiliency for the purpose of providing the cooperative movement relative to the shield and the collar of the assembly.

## Claims

1. An assembly comprising:
a needle assembly comprising a hub (60) and a needle (40) connected to the hub, the needle comprising an intravenous end (44) having a bevel,
a safety shield assembly (140) connected to the hub (60), the safety shield assembly comprising a collar (90) and a shield (140) connected to the collar, the shield comprising a slot (160), wherein the shield is capable of being pivoted from a position at which the intravenous end is exposed to a position at which the intravenous end is shielded within the slot,
**characterized in that**
the collar (90) is affixed to the needle assembly such that the slot is aligned with the bevel up orientation.

2. The assembly of claim 1, wherein the collar (90) comprises a hook arm (114) and the shield (140) comprises a hanger bar (182) that engages the hook arm by an interference fit, thereby connecting the shield to the collar.

3. The assembly of claim 1, wherein the needle is a double needle which comprises a non-patient end (42).

4. The assembly of claim 1, wherein the shield comprises elements (194) that engage cooperating elements (118) located on the collar (90) upon pivoting the shield to the position where the intravenous end (44) is shielded within the slot (160), thereby securing the shield to the collar.
